(19) Europäisches Patentamt
European Patent Office
Office européen des brevets

(11) Publication number: **0 304 282**
**A1**

(12) **EUROPEAN PATENT APPLICATION**

(21) Application number: **88307616.8**

(22) Date of filing: **17.08.88**

(51) Int. Cl.[4]: **C 07 D 403/12**
C 07 D 239/47,
C 07 D 251/16,
C 07 D 401/12,
C 07 D 409/12,
C 07 D 239/52,
C 07 D 239/42,
C 07 D 251/52, A 01 N 47/36

(30) Priority: **19.08.87 US 86867**

(43) Date of publication of application:
**22.02.89 Bulletin 89/08**

(84) Designated Contracting States: **ES GR**

(71) Applicant: **E.I. DU PONT DE NEMOURS AND COMPANY**
**1007 Market Street**
**Wilmington Delaware 19898 (US)**

(72) Inventor: **Chen, Chia-Chung**
**1200 South 47th Street**
**Richmond California 94804 (US)**

**Tocker, Stanley**
**4656 Norwood Drive**
**Wilmington Delaware 19803 (US)**

(74) Representative: **Hildyard, Edward Martin et al**
**Frank B. Dehn & Co. European Patent Attorneys Imperial House 15-19 Kingsway**
**London WC2B 6UZ (GB)**

(54) **Process for preparing sulfonylurea salts.**

(57) A process for preparing salts of herbicidal sulfonylureas comprises contacting a solution of the corresponding sulfonylurea in a water-immiscible halogenated hydrocarbon solvent under reaction conditions with the corresponding alkali metal or alkaline earth metal hydroxide and separating the sulfonylurea salt.

EP 0 304 282 A1

**Description**

## PROCESS FOR PREPARING SULFONYLUREA SALTS

### Related Applications

This application is a continuation-in-part of my copending applications U.S. 86,867 filed August 19, 1987.

### Background of the Invention

U.S. 4,599,412 and U.S. 4,659,823 disclose the preparation of salts of sulfonylureas using a variety of methods, including the reaction of the corresponding N-protonated sulfonylureas with a solution of an alkali or alkaline earth metal salt having a sufficiently basic anion (e.g. hydroxide), or via ion exchange. These methods provide the salt of sulfonylureas with about 70% purity according to elemental analysis. The art process employs water or water-miscible solvents (e.g. methanol, tetrahydrofuran) and hence, results in a less pure salt contaminated with water and must incorporate molecular sieves into the process. The chemical stability of these water-contaminated products is sometimes not sufficient to meet commercial requirements.

### Summary of the Invention

This invention comprises a novel process for the preparation of herbicidal sulfonylurea salts of Formula I by treating a solution of the sulfonylurea in a water-immiscible halogenated hydrocarbon solvent with the appropriate alkali or alkaline earth metal hydroxide. The sulfonylurea salts are then isolated by evaporation of the solvent.

Alternatively, the salts of formula I may be prepared directly in a non-hydroxylic, water-miscible solvent to give a stable 5-40% AI liquid formulation.

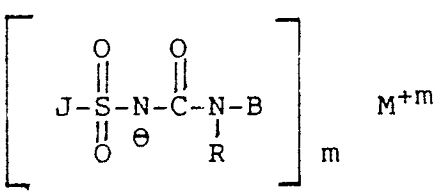

Formula I

wherein:

M is Li, Na, K, Mg or Ca;
R is H or alkyl;
J is an optionally substituted homocyclic group or heterocyclic group;
B is an optionally substituted heterocyclic group whose cyclic skeleton is composed of carbon and at least one nitrogen atom: and
m is 1 or 2

It is understood that when R is H, the salt prepared may consist of an equilibrating pair of deprotonated species as depicted in Scheme 1.

Scheme 1

**POOR QUALITY**

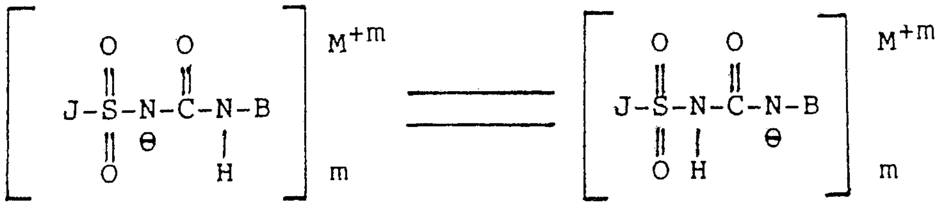

Non-limiting embodiments of processes of the instant invention providing salts preferred for reason of their greater herbicidal activity or greater stability are processes providing salts of Formula I wherein:

R is H or $CH_3$;

J is

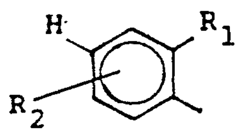 , 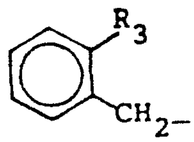 , 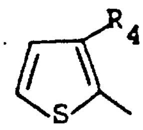 ,

J-1 J-2 J-3

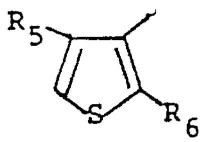 , 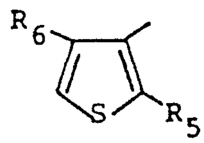 , 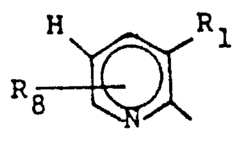 , 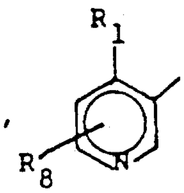

J-4 J-5 J-6 J-7

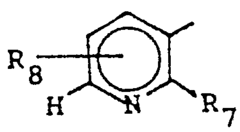 , 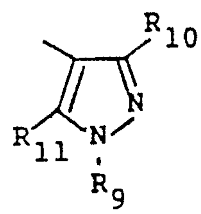 , 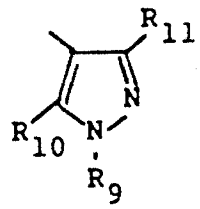 ,

J-8 J-9 J-10

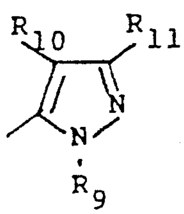 , 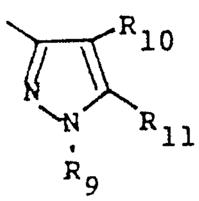 , 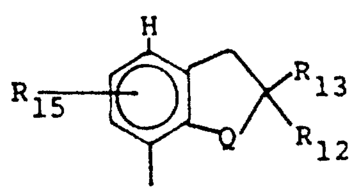 ,

J-11 J-12 J-13

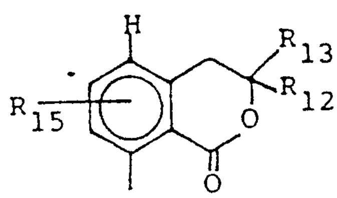 , 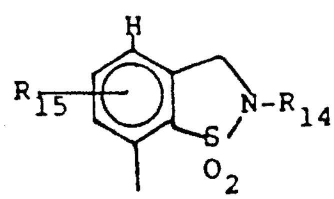 ,

J-14 J-15

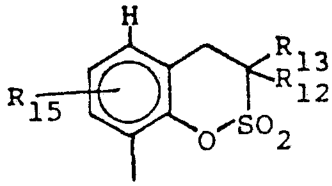 or 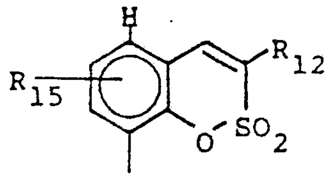 ;

J-16

J-17

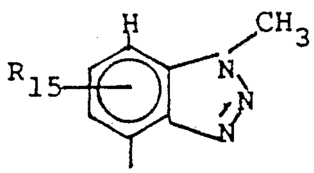

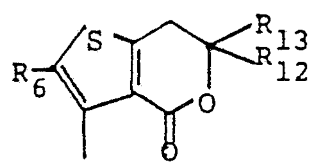

J-18

J-19

wherein

Q is O, S, S(O), $S(O)_2$ or C(O);

$R_1$ is F, Cl, Br, $NO_2$, $C_1$-$C_3$ alkyl, cyclopropyl, $C_1$-$C_3$ haloalkyl, $C_2$-$C_3$ haloalkenyl, $C_1$-$C_4$ alkoxy, $C_1$-$C_3$ haloalkoxy, $C_2$-$C_3$ alkoxyalkoxy, $CO_2R_a$, $C(O)NR_bR_c$, $S(O)_2NR_dR_e$, $S(O)_nR_f$, $OSO_2C_1$-$C_3$ alkyl, $C(O)R_g$, $CH_2CN$ or L;

$R_2$ is H, F, Cl, Br, $NO_2$, CN, $C_1$-$C_3$ alkyl, $CF_3$, $C_1$-$C_4$ alkoxy, $OCF_2H$, $C_2$-$C_3$ haloalkoxy, $C_1$-$C_4$ alkylthio;

$R_3$ is $NO_2$, $CO_2CH_3$, $CO_2CH_2CH_3$, $S(O)_2N(CH_3)_2$, $S(O)_2CH_3$ or $S(O)_2CH_2CH_3$;

$R_4$ is H, F, Cl, Br, $C_1$-$C_3$ alkyl, $C_1$-$C_2$ haloalkyl, $C_2$-$C_3$ haloalkenyl, $C_1$-$C_2$ alkoxy, $NO_2$, $CO_2R_a$, $OSO_2C_1$-$C_3$ alkyl, $C(O)NR_bR_c$, $S(O)_2NR_dR_e$, $S(O)_nR_f$, $C(O)R_g$ or L;

$R_5$ is F, Cl, Br, $C_1$-$C_3$ alkyl, $C_1$-$C_2$ haloalkyl, $C_2$-$C_3$ haloalkenyl, $C_1$-$C_2$ alkoxy, $NO_2$, $CO_2R_a$, $OSO_2C_1$-$C_3$ alkyl, $C(O)NR_bR_c$, $S(O)_2NR_dR_e$, $S(O)_nR_f$, $C(O)R_g$ or L;

$R_6$ is H, F, Cl, Br or $CH_3$;

$R_7$ is F, Cl, Br, $C_1$-$C_3$ alkyl, $C_1$-$C_2$ alkoxy, $C_2$-$C_3$ haloalkenyl, $CO_2R_a$, $C(O)NR_bR_c$, $S(O)_2NR_dR_e$, $S(O)_nR_f$, $C(O)R_g$ or L;

$R_8$ is H, F, Cl, $CH_3$ or $OCH_3$;

$R_9$ is H or $C_1$-$C_2$ alkyl;

$R_{10}$ is F, Cl, Br, $C_1$-$C_3$ alkyl, $C_2$-$C_3$ haloalkenyl, $C_1$-$C_2$ alkoxy, $OCF_2H$, $NO_2$, $CO_2R_a$, $C(O)NR_bR_c$, $S(O)_2NR_dR_e$, $S(O)_nR_f$, $C(O)R_g$ or L;

$R_{11}$ is H, Cl, F, Br, $C_1$-$C_3$ alkyl or $C_1$-$C_2$ alkoxy;

$R_{12}$ is H or $C_1$-$C_2$ alkyl;

$R_{13}$ is H or $CH_3$;

$R_{14}$ is H, $C_1$-$C_6$ alkyl, $C_1$-$C_4$ haloalkyl, $C_2$-$C_4$ alkoxyalkyl or $C_4$-$C_6$ cycloalkylalkyl;

$R_{15}$ is H, F, Cl, Br, $CH_3$, $OCH_3$ or $SCH_3$;

n is 0, 1 or 2;

$R_a$ is $C_1$-$C_3$ alkyl optionally substituted by halogen, $C_1$-$C_2$ alkoxy or CN, cyclopropylmethyl, allyl or propargyl;

$R_b$ is H, $C_1$-$C_3$ alkyl or $C_1$-$C_2$ alkoxy;

$R_c$ is $C_1$-$C_2$ alkyl;

$R_d$ is $C_1$-$C_4$ alkyl, $C_1$-$C_2$ alkoxy, allyl or cyclopropyl;

$R_e$ is H or $C_1$-$C_2$ alkyl;

$R_f$ is $C_1$-$C_3$ alkyl, $C_1$-$C_2$ haloalkyl, allyl or propargyl;

$R_g$ is $C_1$-$C_4$ alkyl, $C_1$-$C_4$ haloalkyl or $C_3$-$C_5$ cycloalkyl;

L is 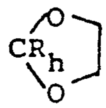 , 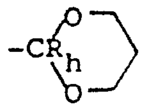 , 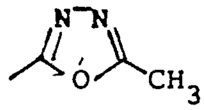 , 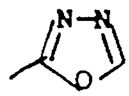 ,

L-1 L-2 L-3 L-4

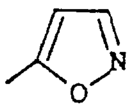 , 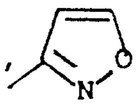 , 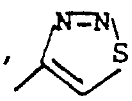 , 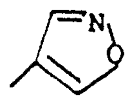 ,

L-5 L-6 L-7 L-8

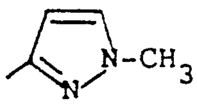 , 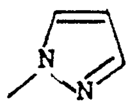 , 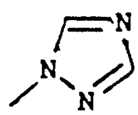 , 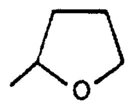 ,

L-9 L-10 L-11 L-12

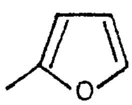 , 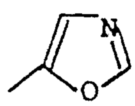 , 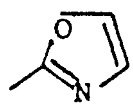 , 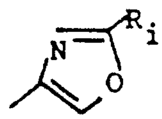 ,

L-13 L-14 L-15 L-16

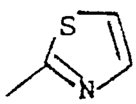 , 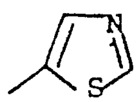 , 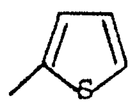 , 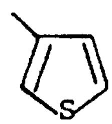 ,

L-17 L-18 L-19 L-20

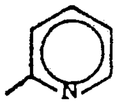 , 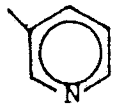 , 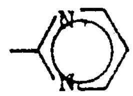 , 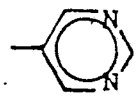 ,

L-21 L-22 L-23 L-24

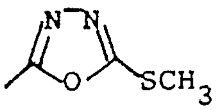 , 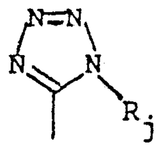 , 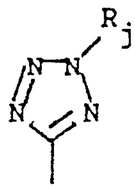 , 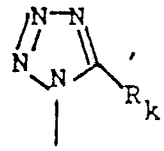 ,

L-25 L-26 L-27 L-28

or 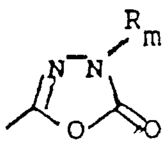 ;

L-29

$R_h$ is H or $CH_3$;
$R_i$ is H or $CH_3$;
$R_j$ is H, $CH_3$ or $CH_2CH_3$;
$R_k$ is H, $CH_3$, $CH_2CH_3$, $OCH_3$, $OCH_2CH_3$, $SCH_3$ or $SCH_2CH_3$;
$R_m$ is H or $CH_3$;
B is

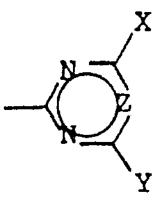

X is $CH_3$, $OCH_3$, $OC_2H_5$, $OCF_2H$ or Cl;
Y is $C_1$-$C_2$ alkyl, $C_1$-$C_2$ alkoxy, $OCF_2H$, $OCH_2CF_3$, $NHCH_3$ or $N(CH_3)_2$; and
Z is CH or N
provided that

1) when X is Cl, then Z is CH and Y is $C_1$-$C_2$ alkoxy and
2) when X or Y is $OCF_2H$, then Z is CH.

In the above definitions, the term "alkyl", used either alone or in compound words such as "alkylthio" or "haloalkyl", denotes straight chain or branched alkyl, e.g., methyl, ethyl, n-propyl, isopropyl or the different butyl isomers.

Alkoxy denotes methoxy, ethoxy, n-propyloxy, isopropyloxy and the different butyloxy isomers.

Alkenyl denotes straight chain or branched alkenes, e.g., vinyl, 1-propenyl, 2-propenyl and 3-propenyl.

Alkylthio denotes methylthio, ethylthio and the different propylthio and butylthio isomers.

Cycloalkyl denotes cyclopropyl, cyclobutyl and cyclopentyl.

$C_4$-$C_6$ cycloalkylalkyl means cyclopropylmethyl through cyclopropylpropyl or cyclopentylmethyl.

The term "halogen", either alone or in compound words such as "haloalkyl", denotes fluorine, chlorine, bromine or iodine. Further, when used in compound words such as "haloalkyl" said alkyl may be partially or fully substituted with halogen atoms, which may be the same or different. Examples of haloalkyl include

$CH_2CH_2F$, $CF_2CF_3$ and $CH_2CHFCl$. The term "haloalkenyl" is defined analogously to the term "haloalkyl".

The total number of carbon atoms in a substituent group is indicated by the $C_i$-$C_j$ prefix where i and j are numbers from 1 to 6. For example, $C_1$-$C_3$ alkylthio would designate methylthio through propylthio; $C_2$ alkoxyalkoxy would designate $OCH_2OCH_3$; $C_3$ alkoxyalkoxy would designate the various isomers of an alkoxy group substituted with a second alkoxy group containing a total of 3 carbon atoms, examples including $OCH_2OCH_2CH_3$, $OCH(CH_3)OCH_3$ and $OCH_2CH_2OCH_3$; as a further example $C_2$ alkoxyalkyl would designate $CH_2OCH_3$ and $C_4$ alkoxyalkyl would designate the various isomers of an alkyl group substituted with an alkoxy group containing a total of 4 carbon atoms, such as $CH_2OCH_2CH_2CH_3$, $CH_2OCH(CH_3)_2$, $CH_2CH_2OCH_2CH_3$, $CH_2CH_2CH_2OCH_3$, $CH(CH_2CH_3)OCH_3$ and $C(CH_3)_2OCH_3$.

Specifically Preferred

Specifically preferred for reasons of higher yield, or greater herbicidal activity or chemical stability of the salts prepared are processes of the instant invention providing either the isolated salt or liquid formulation of the salt;

1. N-[[N-(4,6-dimethoxy-1,3,5-triazin-2-yl)-N-methyl-amino]carbonyl]-2-(5-methyl-1H-tetrazol-1-yl)-benzenesulfonamide, lithium salt. (Compound1)
2. N-[[N-(4,6-dimethoxy-1,3,5-triazin-2-yl)-N-methyl-amino]carbonyl]-2-(5-methyl-1H-tetrazol-1-yl)-benzenesulfonamide, sodium salt.
3. ethyl 2-[[[[(4-chloro-6-methoxy-2-pyrimidinyl)amino]carbonyl]amino]sulfonyl]benzoate, lithium salt. (Compound 2)
4. ethyl 2-[[[[(4-chloro-6-methoxy-2-pyrimidinyl)amino]carbonyl]amino]sulfonyl]benzoate, sodium salt.
5. methyl 2-[[[[N-(4-methoxy-6-methyl-1,3,5-triazin-2-yl)-N-methylamino]carbonyl]amino]sulfonyl]benzoate, lithium salt. (Compound 3)
6. methyl 2-[[[[N-(4-methoxy-6-methyl-1,3,5-triazin-2-yl)-N-methylamino]carbonyl]amino]sulfonyl]benzoate, sodium salt.
7. 2-[[[[(4,6-dimethoxy-2-pyrimidinyl)amino]carbonyl]amino]sulfonyl]-N,N-dimethyl-3-pyridinecarboxamide, lithium salt. (Compound 4)
8. 2-[[[[(4,6-dimethoxy-2-pyrimidinyl)amino]carbonyl]amino]sulfonyl]-N,N-dimethyl-3-pyridinecarboxamide, sodium salt.
9. The liquid formulation of Preferred 5.
10. The liquid formulation of Preferred 6.
11. 2-(2-chloroethoxy)-N-(((((4-methoxy-6-methyl-1,3,5-triazin-2-yl)amino))carbonyl))benzenesulfonamide, lithium salt. (Compound 5)
12. 2-(2-chloroethoxy)-N-(((((4-methoxy-6-methyl-1,3,5-triazin-2-yl)amino))carbonyl))benzenesulfonamide, sodium salt.
13. methyl 3-((((((((4-methoxy-6-methyl-1,3,5-triazin-2-yl)amino))carbonyl))amino))sulfonyl))-2-thiophenecarboxylate, lithium salt. (Compound 6)
14. methyl 3-((((((((4-methoxy-6-methyl-1,3,5-triazin-2-yl)amino))carbonyl))amino))sulfonyl))-2-thiophenecarboxylate, sodium salt.
15. methyl 2-[[[[[(4,6-dimethoxy-2-pyrimidinyl)amino]carbonyl]amino]sulfonyl]methylbenzoate, lithium salt. (Compound 7)
16. methyl 2-[[[[[(4,6-dimethoxy-2-pyrimidinyl)amino]carbonyl]amino]sulfonyl]methylbenzoate, sodium salt.
17. methyl 2-[[[[(4,6-dimethyl-2-pyrimidinyl)amino]carbonyl]amino]sulfonyl]benzoate, lithium salt. (Compound 8)
18. methyl 2-[[[[(4,6-dimethyl-2-pyrimidinyl)amino]carbonyl]amino]sulfonyl]benzoate, sodium salt.
19. 2-chloro-N-[[4-methoxy-6-methyl-1,3,5-triazin-2-yl)amino]carbonyl]amino]benzenesulfonamide, lithium salt. (Compound 9)
20. 2-chloro-N-[[4-methoxy-6-methyl-1,3,5-triazin-2-yl)amino]carbonyl]benzenesulfonamide, sodium salt.
21. methyl 2-[[[[(4-methoxy-6-methyl-1,3,5-triazin-2-yl)amino]carbonyl]amino]sulfonyl]benzoate, lithium salt. (Compound 10)
22. methyl 2-[[[[(4-methoxy-6-methyl-1,3,5-triazin-2-yl)amino]carbonyl]amino]sulfonyl]benzoate, sodium salt.
23. methyl 2-[[[[(4-ethoxy-6-methylamino-1,3,5-triazin-2-yl)amino]carbonyl]amino]sulfonyl]benzoate, lithium salt. (Compound 11)
24. methyl 2-[[[[(4-ethoxy-6-methylamino-1,3,5-triazin-2-yl)amino]carbonyl]amino]sulfonyl]benzoate, sodium salt.
25. ethyl-5-[[[[(4,6-dimethoxy-2-pyrimidinyl)amino]carbonyl]amino]sulfonyl]-1-methyl-1H-4-pyrazolecarboxylate, lithium salt. (Compound 12)
26. ethyl-5-[[[[(4,6-dimethoxy-2-pyrimidinyl)amino]carbonyl]amino]sulfonyl]-1-methyl-1H-4-pyrazolecarboxylate, sodium salt.
27. 2-[[[N-(4-methoxy-6-methyl-1,3,5-triazin-2-yl)N-amino]carbonyl]2-(2-methoxyethoxy)]benzenesulfonamide, lithium salt. (Compound 13)

28. 2-[[[N-(4-methoxy-6-methyl-1,3,5-triazin-2-yl)N-amino]carbonyl]2-(2-methoxyethoxy)]benzenesulfonamide, sodium salt.

29. The liquid formulation of Preferred 10 wherein the solvent is triethylphosphate.

30. The liquid formulation of Preferred 10 wherein the solvent is 1-methyl-2-pyrrolidine.

31. The liquid formulation of Preferred 29 or 30 prepared directly by the one step process for the preparation of the salt in the aprotic organic solvent.

## Detailed Description of the Invention

This invention relates to a novel process for the preparation of sulfonylurea salts for use in liquid and dry flowable formulations with improved chemical stability and biological activity. Such formulations are miscible with water to give true solutions and are useful as preemergent or postemergent herbicides.

Part of this novel process employs water-immiscible halogenated hydrocarbons as solvent in which a heterogeneous reaction between sulfonylurea and metal hydroxide takes place. The process provides very pure salts as products (greater than 96% purity according to elemental analysis). Dry flowable or liquid formulations of salts prepared by this process have excellent storage stability, making them suitable for commercialization.

An alternate process employs a water-miscible solvent wherein a homogeneous reaction between a sulfonylurea and metal hydroxide takes place. The process provides very pure ( 96%) liquid formulations of salts. These formulations have excellent storage stability and are readily water miscible. Examples of water soluble solvents are triethylphosphate, 1-methyl-2-pyrrolidine, diacetone alcohol, dimethylsulfoxide and dimethylformamide.

The free sulfonylureas whose salts are prepared by this process can in turn be prepared by methods known in the art. For example, see U.S. Patents 4,127,405; 4,310,346; 4,370,480; 4,383,113; 4,394,506; 4,398,939; 4,420,325; 4,435,206; 4,441,910; 4,548,638; 4,452,628; 4,456,469; 4,481,029; 4,494,979; 4,544,401; 4,547,215; 4,586,950; 4,643,759; 4,655,817; and 4,659,369; European Patent Applications 44,209, published January 20, 1982; 44,808, published January 27, 1982; 79,683, published May 25, 1983; 83,975, published July 20, 1983; 85,476, published August 10, 1983; 95,925, published December 7, 1983; 155,767, published September 25, 1985; 162,723, published November 27, 1985; 165,753, published December 27, 1985 and 204,513, published December 10, 1986; and South African Patent Applications 82/5042, 81/4874, 82/5045 and 82/5671, the disclosure of which are herein incorporated by reference.

A part of this invention comprises a novel process for the preparation of a sulfonylurea salt by mixing the sulfonylurea and a controlled amount of an alkali or alkaline earth metal hydroxide in a halogenated hydrocarbon solvent. The heterogeneous reaction suspension is stirred for 10 to 16 hours before filtration and evaporation of the solvent to leave the salt.

The heterogeneous reaction of this invention is illustrated schematically in Equation 1.

Equation 1

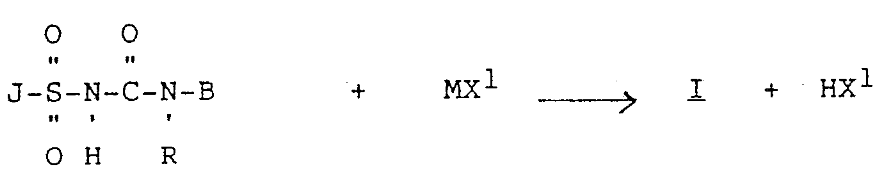

II

wherein

B, J, R and M are as previously described and $MX^1$ is the alkali or alkaline earth metal salt used as base.

Useful bases include alkali or alkaline earth metal hydroxides, methoxides and carbonates, among which sodium hydroxide is preferred because of its low cost and its ability to provide easily characterized products. The base can be used as obtained commercially (e.g., pellet or powder). However, powder form of the base is preferred as it reacts more efficiently due to its greater surface area for contact in this heterogeneous reaction.

The stoichiometry of base is important in this process. For example, while the process can be operated using 1.0 to 1.3 equivalents of sodium hydroxide for every equivalent of technical-grade sulfonylurea (purity ca. 95%), use of 1.1 to 1.2 equivalents of the base is preferred, as it gives a more stable product. Most

preferred is 1.15 to 1.20 equivalents of the base, as it has been found to provide the best product of which the solution in triethyl phosphate is the most stable under the simulated aging test. Table I summarizes the aging test results of various sodium salt products of methyl 2-[[[[N-(4-methoxy-6-methyl-1,3,5-triazin-2-yl)-N-methylamino]carbonyl]amino]sulfonyl]benzoate (Compound 3) prepared by addition of insufficient or excess amounts of sodium hydroxide.

Table I

| Stoichiometry of NaOH | % Recovery of Sodium Salt of Compound 3 in Triethylphosphate After One Week at 55°C |
|---|---|
| 0.5 eq. | 45% |
| 0.9 eq. | 79% |
| 1.1 eq. | 92% |
| 1.2 eq. | 100% |
| 1.5 eq. | 96% |

The data in Table I thus indicates that about 1.15 to 1.20 equivalents is the optimal amount of base. It is believed that the small excess amount of base (ca. 0.15 eq.) is required to quench the acidic contaminants in the technical material of sulfonylurea. Use of less than 1.15 eq. of base results in somewhat incomplete formation of sulfonylurea salt and may provide in liquid formulation a mixture of the parent sulfonylurea and its salt. The parent sulfonylurea decomposes rapidly during the simulated aging test. Any excess base over the amount of 1.15-1.20 eq. may react with the sodium salt of Compound 3 and reduce the chemical yield of the sodium salt of the sulfonylurea.

Useful solvents for this process include methylene chloride, chloroform, trichloroethanes and other halogenated hydrocarbon solvents, provided that the solvents are water-immiscible. Among these solvents, methylene chloride and chloroform are most preferred because of their low cost and their low boiling points which facilitates the removal of solvent after the reaction. Pre-drying of these solvents before the reaction is not essential if the water content of these solvents is below 0.02 wt.%. With wet solvents, drying by stirring the solvent over anhydrous sodium bicarbonate is desirable. The preservative (ethanol, 0.75%) in chloroform is not detrimental to the process as the final step of this process involves evaporation, which efficiently removes ethanol as well as chloroform.

When employing solvents with boiling points greater than about 30°C, the reaction can be run at ambient temperature or at elevated temperature (range 30 to 50°C). When methylene chloride or chloroform is used, ambient temperature reaction is preferred. When fluorotrichloromethane is used as solvent, an ice bath should be used in order to prevent the solvent from evaporating.

The amount of solvent for the reaction is not very critical. Generally, 10 to 40 parts by weight of the solvent is used for every part of sulfonylurea in the reaction. When methylene chloride or chloroform is used as the solvent, 20 parts by weight is preferred to provide an efficient reaction for complete formation of the sulfonylurea salt.

The time needed for the completion of this type of reaction depends on the species of sulfonylurea and the base. When a metal methoxide is used as the base, the reaction typically requires 4 to 10 hours to reach completion. When a metal hydroxide is used as the base, a longer reaction time (10 to 16 hours) is needed. Among all alternatives, a reaction run with sodium hydroxide for a period of 14 to 16 hours is preferred.

Depending on the species of sulfonylurea and solvent, the salt formed during the reaction may or may not be dissolved in the solvent. If the salt is dissolved in the solvent, the solution is filtered, and the filtrate is evaporated at ambient temperature (20-25°C) under reduced pressure (20-30 mm Hg) to give the salt as a solid. If the salt is not dissolved in the solvent, the suspension is filtered and the collected solid is further washed on the filter with fresh solvent (about 10 parts of solvent by weight for every part of sulfonylurea used at the beginning of the reaction).

The addition of a drying agent to the reaction mixture is optional. Suitable drying agents are obvious to one skilled in the art and include alkali metal sulfates, chlorides and their carbonates, among which the alkali metal sulfates are preferred. The amount of drying agent is not critical but one part by weight for every part of sulfonylurea is usually desirable. The addition of the drying agent can be carried out at any time during the

reaction. After the reaction is complete, the drying agent can be removed easily by filtration. When the sulfonylurea salt is not soluble in the solvent, the addition of drying agent should be avoided since it will require an extra step to separate the sulfonylurea salt from the drying agent after the reaction is complete.

The isolated salts of sulfonylurea are often very hygroscopic. The salts should be dried under vacuum (1-30 mm Hg) at ambient temperature and then stored in a desiccator.

The dry salts can be dissolved in water-miscible organic solvents such as triethyl phosphate, 1-methyl-2-pyrrolidinone, diacetone alcohol and dimethyl sulfoxide . The concentration can range from 5% to 30% by weight. Among these solvents, triethyl phosphate and 1-methyl-2-pyrrolidinone are preferred since they provide liquid formulations with the best chemical stability in the simulated aging test. The formulation of the sodium salt of Compound 3 in 1-methyl-2-pyrrolidinone (20%) undergoes less than 2% decomposition after one week at 55°C. The solution of same salt in triethyl phosphate (20%) is also very chemically stable (2% decomposition after three weeks at 55°C).

An alternative approach as already described is to prepare the stable highly pure salt of the sulfonylurea directly in a solvent suitable for agricultural formulations. This avoids the necessity of isolating the sulfonylurea salt and then adding said salt to the appropriate water miscible solvent.

For example, addition of a hydroxide salt such as NaOH or KOH to a suspension of a compound of Formula I in triethylphosphate results in a water miscible stable formulation of the salt of compound of Formula I in triethylphosphate

Suitable salts and solvents are as previously described. The typical stable formulations can range from 5-40%. Typical reaction temperatures may range from 0 to 60°C with ambient temperatures being preferred. Typical reaction times will range from 1 hour to 2 days with 1 day being preferred. The solvents employed may be dried prior to their use. Typical drying conditions are 4A molecular sieves for 24 hours.

The liquid formulations prepared as above, by either the two-step process or prepared directly insitu via the described one-step process are applicable to all salts of Formula I. However, long term liquid stability has been observed only when R is alkyl.

The organic solvents for the formulation can be used as obtained commercially if the water content in these solvents is less than 0.5%. Otherwise, it is desirable to dry the solvents by conventional methods. Many of the solvents can be dried over anhydrous magnesium sulfate followed by filtration to remove the drying agent. The solvent should then be stored in a sealed bottle or over molecular sieves. This drying procedure is not applicable for dimethyl sulfoxide, which should instead be dried by the conventional method (distill at reduced pressure from NaOH pellets and store over 4A molecular sieves).

These liquid formulations can tolerate the presence of small amounts of hydroxylated impurities (e.g., 2% content of 1-propanol) without significant reduction of their stability. A 0.5% content of water in the liquid formulation, for instance, does not reduce the chemical stability of the liquid.

The dry salt itself is chemically very stable. In the Simulated Aging Test, below, the dry salt does not break down at an observable rate. The dry salt can be used to prepare dry flowable formulations or it can be used directly as a pre- or postemergent herbicide.

Both the isolated salts and their liquid formulation are completely water soluble. They form clear, true solutions when mixed with water in a spray tank at commercial concentrations of 100 to 10,000 ppm. Aqueous solutions of these salts may be stored in a spray tank for at least 10 hours without significant breakdown. The pH value of water is preferably from 7.0 to 8.0, as the weakly basic water dissolves the salt of sulfonylurea more rapidly and efficiently than neutral or acidic water.

## Experimental Procedures

### Simulated Aging Test

The Simulated Aging Test involves heating a sample of the formulation mixture being evaluated at 55°C for a period of time, typically one to three weeks. At the end of this time, the amount of active ingredient remaining is assayed by HPLC (high performance liquid chromatography). One week in the Simulated Aging Test approximates the effect of storage at ambient temperatures for one year.

### High Performance Liquid Chromatography

High Performance Liquid Chromatography (HPLC) was used to determine the amount of the sulfonylurea salt present in the isolated products and in Simulated Aging Test samples. A mixture of acetonitrile and water buffered to pH 2.2 was used as the eluent. One of two columns were used as the stationary phase: Du Pont ZORBAX* SIL ODS 4, 15 cm and Whatman ODS 3 RAC II, 10 cm.

Since the eluent was buffered to pH 2.2, the sulfonylurea salts were reprotonated on the column to the parent sulfonylureas. As these conditions do not differentiate the initial presence of salt from the presence of free sulfonylurea, the utility of HPLC was confined to monitoring for decomposition. The conversion of free sulfonylurea to its salt was confirmed instead by melting point measurement and nuclear magnetic resonance

spectroscopy.

Differential Scanning Calorimetry

In the experimental descriptions, DSC refers to differential scanning calorimetry and was used to determine the melting points of the isolated sulfonylurea salts.

Nuclear Magnetic Resonance Spectroscopy

Nuclear magnetic resonance (NMR) spectra were measured on a Varian EM390 spectrometer. Chemical shifts are reported in parts per million downfield from tetramethylsilane, using the following abbreviations: s, singlet; d, doublet; t, triplet; q, quartet; m, multiplet and J, coupling constant.

COMPOUNDS 1-16

Compound 1

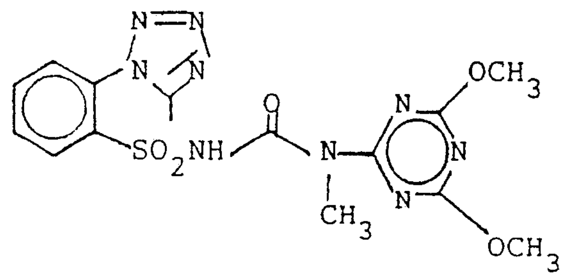

Compound 2

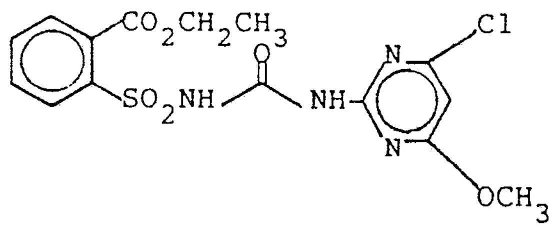

Compound 3

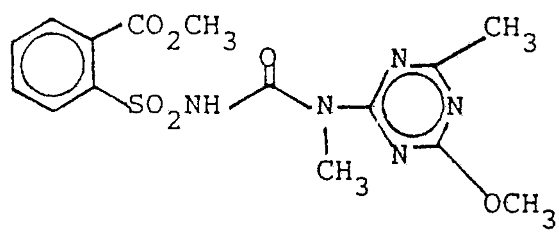

Compound 4

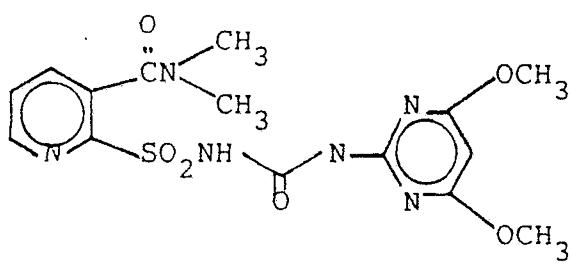

COMPOUNDS 1-16 (continued)

Compound 5

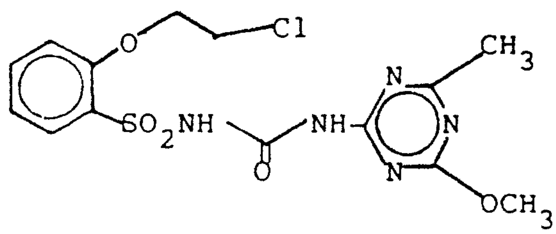

Compound 6

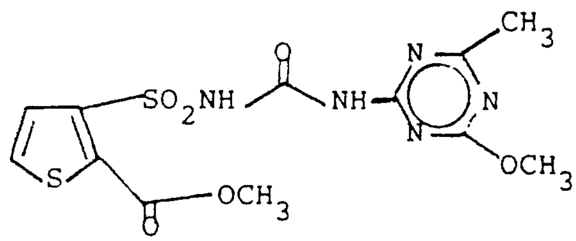

Compound 7

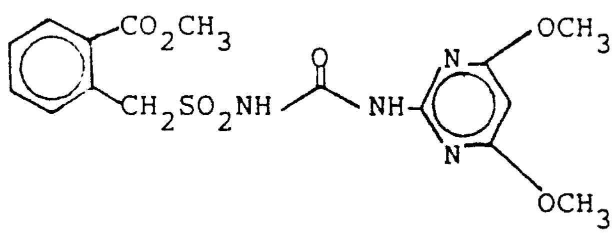

Compound 8

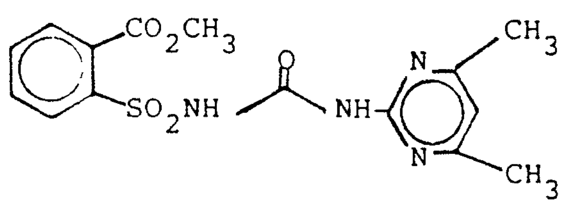

COMPOUNDS 1-16 (continued)

Compound 9

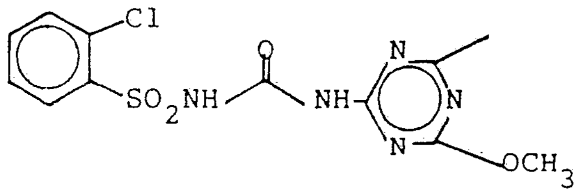

Compound 10

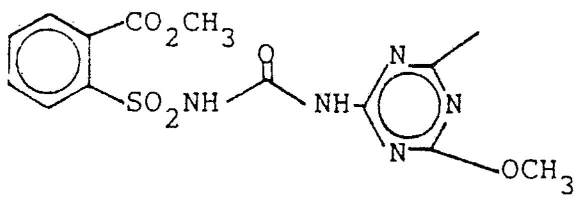

Compound 11

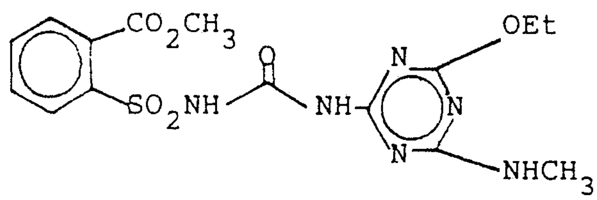

Compound 12

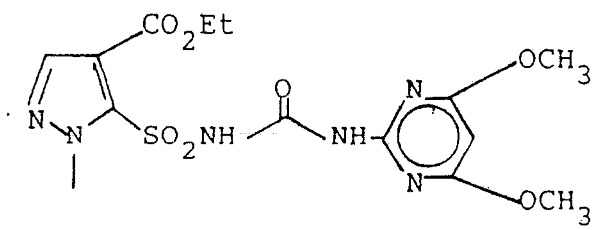

COMPOUND 1-16 (continued)

Compound 13

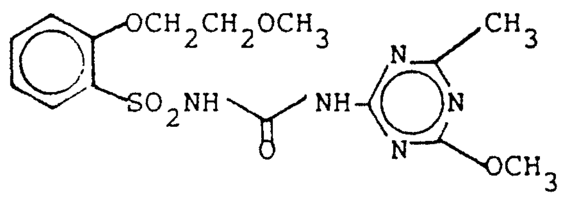

Compound 14

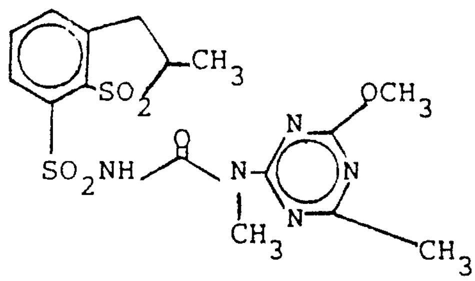

Compound 15

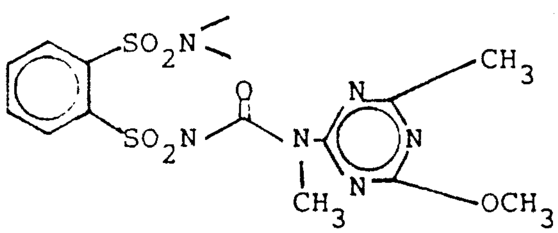

## Examples

The following examples further illustrate the process of this invention.

### Example 1

Preparation of the sodium salt of methyl-2-[[[[N-(4-methoxy-6-methyl-1,3,5-triazin-2-yl)-N-methylamino]carbonyl]amino]sulfonyl]benzoate (Compound 3)

To a stirred suspension of Compound 3 which may be made by methods known in the art (purity 95.7%, 20 g, 48.5 mmol) and sodium carbonate (30 g) in methylene chloride (400 g), was added 2.23 g of sodium hydroxide (powder, 55.7 mmol). The suspension was allowed to stir at ambient temperature (20-25°C) for 16 hours before it was filtered. The filtrate, obtained as a clear yellow solution, was evaporated under reduced pressure using a rotary evaporator. A pale yellow solid (99%) was isolated as the sodium salt of Compound 1 (20 g), m.p. 184°C (DSC). HPLC using 43% acetonitrile as eluent regenerated Compound 3 and gave a retention time ($R_f$) of 7 minutes.

NMR ($CDCl_3$): 8.20-8.05 (m, 1H);
7.47-7.23 (m, 3H);
3.80-3.65 (m, 6H);
3.25 (s, 3H);
2.30 (s, 3H).

Example 2

Preparation of the sodium salt of ethyl 2-[[[[(4-chloro-6-methoxy-2-pyrimidinyl)amino]carbonyl]amino]sulfonyl]benzoate (Compound 2)

Method A

To a stirred solution of Compound 9, which may be made by methods known in the art, (50 g, 0.12 mol) in methylene chloride (1500 g) was added 5.31 g of sodium hydroxide (powder, 0.133 mol). The suspension was stirred at ambient temperature (20-25° C) for 20 hours. The turbid suspension was then filtered, and the solid collected on the filter was further washed twice with fresh methylene chloride. The salt was identified as sodium salt of Compound 2, m.p. 205° C (DSC). HPLC using 45% acetonitrile as eluent gave a $R_f$ of 13 minutes, identical to the retention time of the starting sulfonylurea.
NMR (DMSO-$d_6$): 8.99 (s, 1H);
8.13-8.00 (m, 1H);
7.65-7.40 (m, 3H);
6.45 (s, 1H);
4.30 (q, 2H, J=7 Hz);
3.88 (s, 3H);
1.27 (t, 3H, J=7 Hz).

Method B

A stirred solution of Compound 2 (1 g, 2.4 mmol) in chloroform (50 g) was reacted with sodium hydroxide (0.1 g, 2.5 mmol). The suspension was then stirred at ambient temperature for 16 hours. The slightly turbid solution was filtered to give a clear solution which was evaporated on a rotary evaporator at reduced pressure to give the salt as a yellow solid. The salt obtained by this method had the same NMR spectrum as that obtained in Method A.

Example 3

Preparation of the lithium salt of Compound 2

By using the procedure exemplified in Method A, in Example 2, the lithium salt of Compound 2 was synthesized. DSC measurement showed the lithium salt to melt at 143°C.
NMR (DMSO-$d_6$): 9.12 (s, 1H);
8.13-8.00 (m, 1H);
7.67-7.33 (m, 3H);
6.58 (s, 1H);
4.25 (t, 2H, J=7 Hz);
3.87 (s, 3H);
1.25 (q, 3H, J=7 Hz).

Example 4

Preparation of the lithium salt of 2,3-dihydro-N-[[(4-methoxy-6-methyl-1,3,5-triazin-2-yl)-N-methylamino]carbonyl]-2-methylbenzo[B]thiophene-7-sulfonamide-1,1-dioxide (Compound 14)

By following the general procedure described in Example 1, Compound 3, which may be made by methods known in the art, (0.5 g, 1.13 mmol), lithium carbonate (1 g), lithium hydroxide monohydrate (0.052 g, 1.24 mmol) and methylene chloride (50 g) were used to generate the lithium salt of Compound 14 (0.5 g, 99% yield), m.p. 125°C (dec., DSC). HPLC using 30% acetonitrile as eluent gave a $R_f$ of 6.7 minutes, identical to the retention time of the starting sulfonylurea.

## Example 5

### Preparation of the sodium salt of Compound 14

By following the general procedure described in Example 2 (Method A), Compound 14 (0.5 g, 1.13 mmol), sodium hydroxide (0.05 g, 1.25 mmol) and methylene chloride (50 g) were used to produce the sodium salt of Compound 3 (0.52 g, 99% yield), m.p. 238°C (dec., DSC).

NMR ($DMSO-d_6$): 8.20-8.03 (m, 1H);
7.85-7.53 (m, 2H);
3.87 (s, 3H);
3.55-3.40 (m, 1H);
3.28 (s, 3H);
2.90-2.50 (m, 2H);
2.32 (s, 3H);
1.33 (d, 3H, J=6 Hz).

## Example 6

### Preparation of the sodium salt of N-[[N-4-methoxy-6-methyl-1,3,5-triazin-2-yl)-N-methylamino]carbonyl]-N',N'-dimethyl-1,2-benzenedisulfonamide (Compound 15)

By following the procedure exemplified in Example 2, Method A, Compound 4, which may be made by methods known in the art, (0.283 g, 0.6 mmol), sodium hydroxide (0.028 g, 0.7 mmol) and methylene chloride (28 g) were used to produce the sodium salt of Compound 15 (0.3 g, 0.6 mmol, 100% yield), m.p. 160°C (dec., DSC).

NMR ($CDCl_3$): 8.60-8.40 (m, 1H);
7.85-7.55 (m, 3H);
3.87 (s, 3H);
3.47 (s, 3H);
2.90 (s, 6H);
2.39 (s, 3H);

## Example 7

### Preparation of the lithium salt of Compound 15

By following the procedure exemplified in Method A of Example 2, Compound 4 (0.25 g, 0.56 mmol), lithium hydroxide monohydrate (0.027 g, 0.64 mmol) and methylene chloride (32 g) were used to produce the lithium salt of Compound 15 (0.26 g, 0.57 mmol, 100% yield), m.p. 130°C (dec., DSC).

NMR ($CDCl_3$): 8.45-8.31 (m, 1H);
7.95-7.50 (m, 3H);
3.94 (s, 3H);
3.63 (s, 3H);
2.84 (s, 6H);
2.25 (s, 3H);

## Example 8

Preparation of the sodium salt of N-[[N-4,6-dimethoxy-1,3,5-triazin-2-yl)-N-methylamino]carbonyl]-2-(5-methyl-1H-tetrazol-1-yl)benzenesulfonamide (Compound 1)

By following the procedure exemplified in Example 1, Compound 5, which may be made by methods known in the art, (16.87 g, 38.7 mmol), sodium carbonate (36 g), sodium hydroxide (1.79 g, 44.7 mmol) and methylene chloride (480 g) were used to produce the sodium salt of Compound 16 (17.8 g, 38.7 mmol, 100% yield).
NMR ($CDCl_3$): 8.4-8.13 (m, 1H);
7.70-7.40 (m, 2H);
7.25-7.00 (m, 1H);
3.85 (s, 6H);
3.19 (s, 3H);
2.32 (s, 3H);

Example 9

Preparation of the lithium salt of Compound 1

By following the procedure exemplified in Example 1, Compound 5 (0.5 g 1.1 mmol), lithium hydroxide monohydrate (0.053 g, 1.26 mmol) and methylene chloride (50 g) were used to produce the lithium salt of Compound 16 (0.45 g, 1.02 mmol, 89% yield).
NMR ($CDCl_3$): 8.55-8.20 (m, 1H);
7.90-7.50 (m, 2H);
7.50-7.15 (m, 1H);
3.90 (s. 6H);
3.43 (s, 3H);
2.50 (s, 3H);

Example 10

Preparation of the sodium salt of 2-[[[[(4,6-dimethoxy-2-pyrimidinyl)amino]carbonyl]amino]sulfonyl]-N,N-dimethyl-3-pyridinecarboxamide (Compound 4)

By following the procedure exemplified in Example 1, Compound 4, which may be made by methods known in the art, (5 g, 12.2 mmol), sodium carbonate (5 g), sodium hydroxide (0.56 g, 13.4 mmol) and methylene chloride (150 g) were used to quantitatively produce the sodium salt of Compound 4, m.p. 200°C (dec., DSC).
NMR ($CDCl_3$): 8.40-8.27 (m, 1H);
7.97 (s, 1H);
7.63-7.47 (m, 1H);
7.40-7.17 (m, 1H);
5.54 (s, 1H);
3.77 (s, 6H);
3.10 (s, 3H);
2.81 (s, 3H).

Example 11

Preparation of the sodium salt of methyl 2-[[[[(4-methoxy-6-methyl-1,3,5-triazin-2-yl]amino]carbonyl]amino]sulfonyl]benzoate (Compound 10)

By Method A of Example 2, Compound 10, which may be made by methods known in the art, (5 g, 13.1 mmol), sodium hydroxide (0.57 g, 14.3 mmol) and methylene chloride (150 g) were used to quantitatively produce the sodium salt of Compound 10, m.p. 195°C (DSC).

NMR (DMSO-$d_6$): 9.18 (s, 1H);
8.20-8.07 (m, 1H);
7.70-7.43 (m, 3H);
3.87 (s, 3H);
3.80 (s, 3H);
2.31 (s, 3H).

Example 12

Preparation of the lithium salt of Compound 10

By following the procedure exemplified in Example 1, Compound 10 (3 g, 7.9 mmol), lithium carbonate (3 g), lithium hydroxide monohydrate (0.36 g, 8.6 mmol) and methylene chloride were used to quantitatively produce the lithium salt of Compound 10.
NMR (DMSO-$d_6$): 9.60 (s, 1H);
8.15-7.96 (m, 1H);
7.78-7.37 (m, 3H);
3.90 (s, 3H);
3.76 (s, 3H);
2.32 (s, 3H).

Example 13

Preparation of the sodium salt of methyl 3-[[[[(4-methoxy-6-methyl-1,3,5-triazin-2-yl)amino]carbonyl]amino]sulfonyl]-2-thiophenecarboxylate (Compound 6)

By Method A of Example 2, Compound 6, which may be made by methods known in the art, (5 g, 12.9 mmol), sodium hydroxide (powder, 0.57 g, 14.2 mmol) and methylene chloride (150 g) were used to quantitatively produce the sodium salt of Compound 6, m.p. 200°C (dec., DSC).
NMR (DMSO-$d_6$): 7.87 (d, 1H, J=5 Hz);
7.55 (d, 1H, J=5 Hz);
3.90 (s, 3H);
3.83 (s, 3H);
2.35 (s, 3H).

Direct Preparation of Sulfonylurea Salt Solutions

Example 14

Preparation of a liquid formulation of sodium salt of methyl-2-[[[[N-(4-methoxy-6-methyl-1,3,5-triazin-2-yl)-N-methylamino]carbonyl]amino]sulfonyl]benzoate (Compound 3)

To a stirred suspension of 10 g (24.2 mmoles) of Compound 3 (as the free acid) (95.7% pure) in 55 g triethyl phosphate (predried by standing over 4A molecular sieves 24 hours) was added 1.25 g (31.25 mmoles) of powdered sodium hydroxide. The heterogeneous mixture was magentically stirred in a closed container for 24 hours and was then filtered, giving a pale yellow solution containing 14.4% of the active component. The solution was miscible in all proportions with water.

Example 15

methyl-2-[[[[N-(4-methoxy-6-methyl-1,3,5-triazin-2-yl)-N-methylamino]carbonyl]amino]sulfonyl]benzoate (Compound 3)

The process of Example 14 was repeated using 1.0 g (25 mmoles) sodium hydroxide. A similar product was obtained.

## Example 16

Using a process similar to that described in Example 14, the lithium salt of Compound 3 was prepared in triethylphosphate solution by use of 0.74 g (31.25 mmoles) anhydrous lithium hydroxide instead of sodium hydroxide. The resultant yellow solution contained 14.55% of Compound 3.

## Example 17

The process of Example 16 was repeated using 21 g 1-methyl-2-pyrrolidone instead of triethylphosphate as solvent. The product contained 30.1% of Compound 3 in the form of the lithium salt.

## Example 18

The process of Example 14 was repeated using the mixed solvent of 35 g triethylphosphate and 8 g 1-methyl-2-pyrrolidone. The product contained 17.6% of Compound 3.

The salts prepared by processes 1-13 were dissolved in either triethylphosphate or 1-methyl-2-pyrrolidone or mixtures of these solvents to give solutions containing 1-40% active ingredient that were dissolvable in all proportions with water.

In general, the lithium salts were more soluble than sodium salts. Also, 1-methyl-2-pyrrolidone dissolved at least 25% more salt than triethyl phosphate.

Such solution formulation substantially instantly dissolved in agitated water upon dilution prior to application by spraying. Standard perticulate formulations of the aforementioned compounds required time to dissolve or disperse in water.

## Claims

1. A process for preparing herbicidal sulfonylurea salts of Formula I

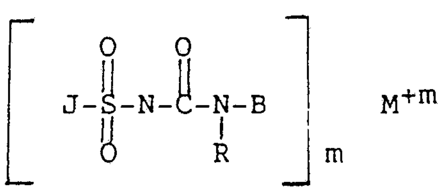

Formula I

wherein:
M is Li, Na, K, Mg or Ca;
R is H or alkyl;
J is an optionally substituted homocyclic group or heterocyclic group;
B is an optionally substituted heterocyclic group whose cyclic skeleton is composed of carbon and at least one nitrogen atom; and
m is 1 or 2
by contacting a solution of the corresponding sulfonylurea in a water-immiscible halogenated hydrocarbon solvent under reaction conditions with the corresponding alkali or alkaline earth metal

hydroxide and separating the sulfonylurea salt.

2. The process of Claim 1 wherein the separation takes place by evaporation.

3. A process for the preparation of a solution of a sulfonylurea salt of the <u>Formula I</u>.

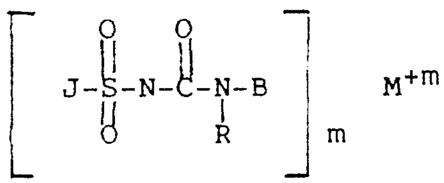

<u>Formula I</u>

wherein:

M is Li, Na, K, Mg or Ca;

R is H or alkyl;

J is an optionally substituted homocyclic group or heterocyclic group;

B is an optionally substituted heterocyclic group whose cyclic skeleton is composed of carbon and at least one nitrogen atom; and

m is 1 or 2

by contacting the corresponding sulfonylurea with an alkaline or alkaline earth base in a water-miscible aprotic solvent.

4. The process of Claim 3 wherein:

R is H or $CH_3$;

J is

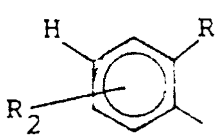 , 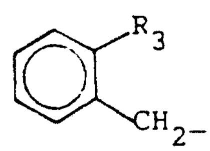 , 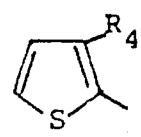 ,

<u>J-1</u> <u>J-2</u> <u>J-3</u>

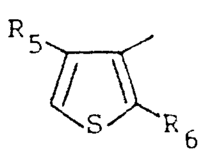 , 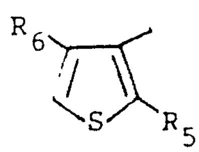 , 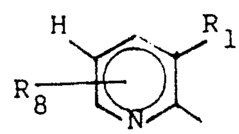 , 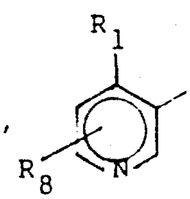

<u>J-4</u> <u>J-5</u> <u>J-6</u> <u>J-7</u>

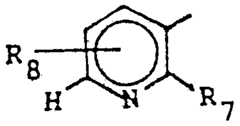 ,

J-8

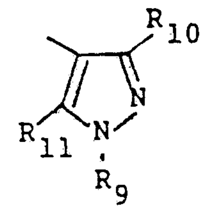 ,

J-9

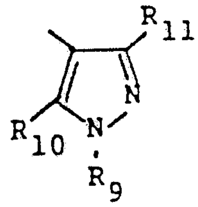 ,

J-10

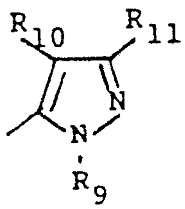 ,

J-11

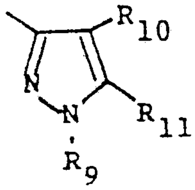 ,

J-12

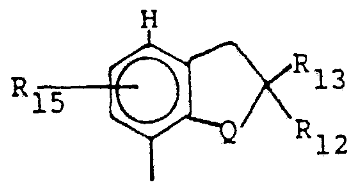 ,

J-13

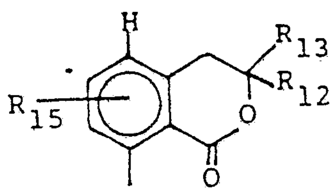 ,

J-14

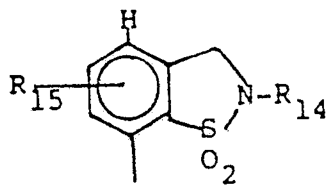 ,

J-15

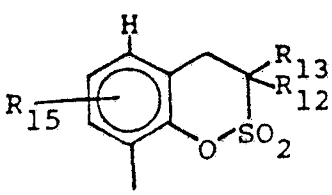 or

J-16

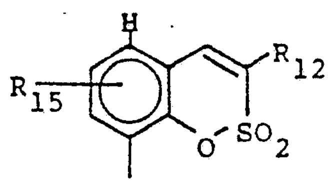 ;

J-17

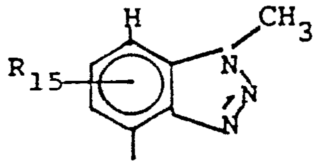

J-18

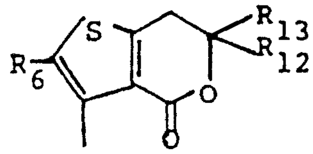

J-19

wherein
Q is O, S, S(O), $S(O)_2$ or C(O);
$R_1$ is F, Cl, Br, $NO_2$, $C_1$-$C_3$ alkyl, cyclopropyl, $C_1$-$C_3$ haloalkyl, $C_2$-$C_3$ haloalkenyl, $C_1$-$C_4$ alkoxy, $C_1$-$C_3$ haloalkoxy, $C_2$-$C_3$ alkoxyalkoxy, $CO_2R_a$, $C(O)NR_bR_c$, $S(O)_2NR_dR_e$, $S(O)_nR_f$, $OSO_2C_1$-$C_3$ alkyl, $C(O)R_g$, $CH_2CN$ or L;
$R_2$ is H, F, Cl, Br, $NO_2$, CN, $C_1$-$C_3$ alkyl, $CF_3$, $C_1$-$C_4$ alkoxy, $OCF_2H$, $C_2$-$C_3$ haloalkoxy, $C_1$-$C_4$ alkylthio;
$R_3$ is $NO_2$, $CO_2CH_3$, $CO_2CH_2CH_3$, $S(O)_2N(CH_3)_2$, $S(O)_2CH_3$ or $S(O)_2CH_2CH_3$;
$R_4$ is H, F, Cl, Br, $C_1$-$C_3$ alkyl, $C_1$-$C_2$ haloalkyl, $C_2$-$C_3$ haloalkenyl, $C_1$-$C_2$ alkoxy, $NO_2$, $CO_2R_a$, $OSO_2C_1$-$C_3$ alkyl, $C(O)NR_bR_c$, $S(O)_2NR_dR_e$, $S(O)_nR_f$, $C(O)R_g$ or L;
$R_5$ is F, Cl, Br, $C_1$-$C_3$ alkyl, $C_1$-$C_2$ haloalkyl, $C_2$-$C_3$ haloalkenyl, $C_1$-$C_2$ alkoxy, $NO_2$, $CO_2R_a$, $OSO_2C_1$-$C_3$ alkyl, $C(O)NR_bR_c$, $S(O)_2NR_dR_e$, $S(O)_nR_f$, $C(O)R_g$ or L;
$R_6$ is H, F, Cl, Br or $CH_3$;
$R_7$ is F, Cl, Br, $C_1$-$C_3$ alkyl, $C_1$-$C_2$ alkoxy, $C_2$-$C_3$ haloalkenyl, $CO_2R_a$, $C(O)NR_bR_c$, $S(O)_2NR_dR_e$, $S(O)_nR_f$, $C(O)R_g$ or L;
$R_8$ is H, F, Cl, $CH_3$ or $OCH_3$;
$R_9$ is H or $C_1$-$C_2$ alkyl;
$R_{10}$ is F, Cl, Br, $C_1$-$C_3$ alkyl, $C_2$-$C_3$ haloalkenyl, $C_1$-$C_2$ alkoxy, $OCF_2H$, $NO_2$, $CO_2R_a$, $C(O)NR_bR_c$, $S(O)_2NR_dR_e$, $S(O)_nR_f$, $C(O)R_g$ or L;
$R_{11}$ is H, Cl, F, Br, $C_1$-$C_3$ alkyl or $C_1$-$C_2$ alkoxy;
$R_{12}$ is H or $C_1$-$C_2$ alkyl;
$R_{13}$ is H or $CH_3$;
$R_{14}$ is H, $C_1$-$C_6$ alkyl, $C_1$-$C_4$ haloalkyl, $C_2$-$C_4$ alkoxyalkyl or $C_4$-$C_6$ cycloalkylalkyl;
$R_{15}$ is H, F, Cl, Br, $CH_3$, $OCH_3$ or $SCH_3$;
n is 0, 1 or 2;
$R_a$ is $C_1$-$C_3$ alkyl optionally substituted by halogen, $C_1$-$C_2$ alkoxy or CN, cyclopropylmethyl, allyl or propargyl;
$R_b$ is H, $C_1$-$C_3$ alkyl or $C_1$-$C_2$ alkoxy;
$R_c$ is $C_1$-$C_2$ alkyl;
$R_d$ is $C_1$-$C_4$ alkyl, $C_1$-$C_2$ alkoxy, allyl or cyclopropyl;
$R_e$ is H or $C_1$-$C_2$ alkyl;
$R_f$ is $C_1$-$C_3$ alkyl, $C_1$-$C_2$ haloalkyl, allyl or propargyl;
$R_g$ is $C_1$-$C_4$ alkyl, $C_1$-$C_4$ haloalkyl or $C_3$-$C_5$ cycloalkyl;

L is 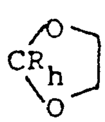 , 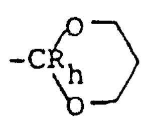 , 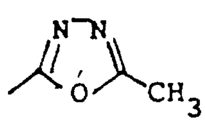 , 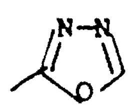 ,

L-1 L-2 L-3 L-4

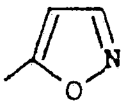 , 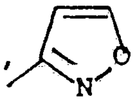 , 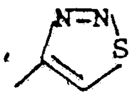 , 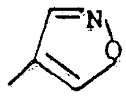 ,

L-5 L-6 L-7 L-8

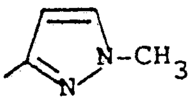 , 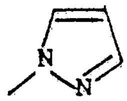 , 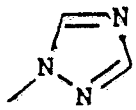 , 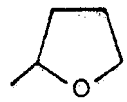 ,

L-9 L-10 L-11 L-12

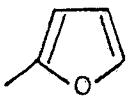 , 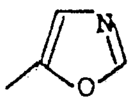 , 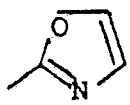 , 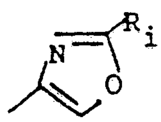 ,

L-13 L-14 L-15 L-16

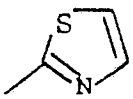 , 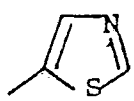 , 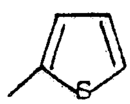 , 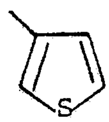 ,

L-17 L-18 L-19 L-20

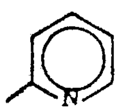 , 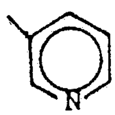 , 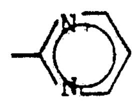 , 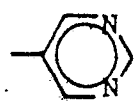 ,

L-21 L-22 L-23 L-24

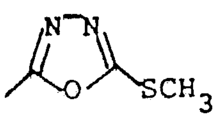 , 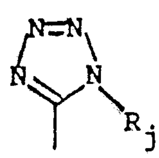 , 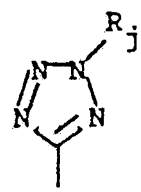 , 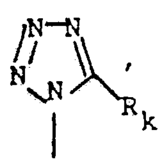 ,

L-25 L-26 L-27 L-28

or 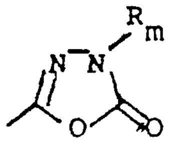 ;

L-29

$R_h$ is H or $CH_3$;
$R_i$ is H or $CH_3$;
$R_j$ is H, $CH_3$ or $CH_2CH_3$;
$R_k$ is H, $CH_3$, $CH_2CH_3$, $OCH_3$, $OCH_2CH_3$, $SCH_3$ or $SCH_2CH_3$;
$R_m$ is H or $CH_3$;
B is

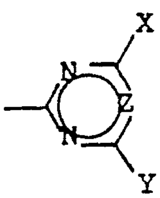

X is $CH_3$, $OCH_3$, $OC_2H_5$, $OCF_2H$ or Cl;
Y is $C_1$-$C_2$ alkyl, $C_1$-$C_2$ alkoxy, $OCF_2H$, $OCH_2CF_3$, $NHCH_3$ or $N(CH_3)_2$; and
Z is CH or N
provided that

1) when X is Cl, then Z is CH and Y is $C_1$-$C_2$ alkoxy and
2) when X or Y is $OCF_2H$, then Z is CH.

5. The process of Claim 1 wherein the compound of Formula I is:
methyl 2-[[[[N-(4-methoxy-6-methyl-1,3,5-triazin-2-yl)-N-methylamino]carbonyl]amino]sulfonyl]benzoate, sodium salt.
6. The process of Claim 3 wherein the compound of Formula I is:
N-[[N-(4,6-dimethoxy-1,3,5-triazin-2-yl)-N-methyl-amino]carbonyl]-2-(5-methyl-1H-tetrazol-1-yl)-benzensulfonamide, sodium salt.
7. The process of Claim 3 wherein the compound of Formula I is:
ethyl 2-[[[[(4-chloro-6-methoxy-2-pyrimidinyl)amino]carbonyl]amino]sulfonyl]benzoate, lithium salt.
8. The process of Claim 3 wherein the compound of Formula I is:
ethyl 2-[[[[(4-chloro-6-methoxy-2-pyrimidinyl)amino]carbonyl]amino]sulfonyl]benzoate, sodium salt.
9. The process of Claim 3 wherein the compound of Formula I is:
methyl 2-[[[[N-(4-methoxy-6-methyl-1,3,5-triazin-2-yl)-N-methylamino]carbonyl]amino]sulfonyl]benzoate, lithium salt.
10. The process of Claim 3 wherein the compound of Formula I is:
methyl 2-[[[[N-(4-methoxy-6-methyl-1,3,5-triazin-2-yl)-N-methylamino]carbonyl]amino]sulfonyl]benzoate, sodium salt.
11. The process of Claim 3 wherein the compound of Formula I is:
2-[[[[(4,6-dimethoxy-2-pyrimidinyl)amino]carbonyl]amino]sulfonyl]-N,N-dimethyl-3-pyridinecarboxamide, lithium salt.
12. The process of Claim 3 wherein the compound of Formula I is:
2-[[[[(4,6-dimethoxy-2-pyrimidinyl)amino]carbonyl]amino]sulfonyl]-N,N-dimethyl-3-pyridinecarboxamide, sodium salt.
13. The process of Claim 3 wherein the compound of Formula I is:
2-(2-chloroethoxy)-N-(((((4-methoxy-6-methyl-1,3,5-triazin-2-yl)amino))carbonyl))benzenesulfonamide, lithium salt.
14. The process of Claim 3 wherein the compound of Formula I is:
2-(2-chloroethoxy)-N-(((((4-methoxy-6-methyl-1,3,5-triazin-2-yl)amino))carbonyl))benzenesulfonamide, sodium salt.
15. The process of Claim 3 wherein the compound of Formula I is:
methyl 3-((((((((4-methoxy-6-methyl-1,3,5-triazin-2-yl)amino))carbonyl))amino))sulfonyl))-2-thiophenecarboxylate, lithium salt.

16. The process of Claim 3 wherein the compound of Formula I is:
methyl 3-((((((((4-methoxy-6-methyl-1,3,5-triazin-2-yl)amino))carbonyl))amino))sulfonyl))-2-thiophenecarboxylate, sodium salt.

17. The process of Claim 3 wherein the compound of Formula I is:
methyl 2-[[[[[(4,6-dimethoxy-2-pyrimidinyl)amino]carbonyl]amino]sulfonyl]methylbenzoate, lithium salt.

18. The process of Claim 3 wherein the compound of Formula I is:
methyl 2-[[[[[(4,6-dimethoxy-2-pyrimidinyl)amino]carbonyl]amino]sulfonyl]methylbenzoate, sodium salt.

19. The process of Claim 3 wherein the compound of Formula I is:
methyl 2-[[[[(4,6-dimethyl-2-pyrimidinyl)amino]carbonyl]amino]sulfonyl]benzoate, lithium salt.

20. The process of Claim 3 wherein the compound of Formula I is:
methyl 2-[[[[(4,6-dimethyl-2-pyrimidinyl)amino]carbonyl]amino]sulfonyl]benzoate, sodium salt.

21. The process of Claim 3 wherein the compound of Formula I is:
2-chloro-N-[[4-methoxy-6-methyl-1,3,5-triazin-2-yl)amino]carbonyl]amino]benzenesulfonamide, lithium salt.

22. The process of Claim 3 wherein the compound of Formula I is:
2-chloro-N-[[4-methoxy-6-methyl-1,3,5-triazin-2-yl)amino]carbonyl]benzenesulfonamide, sodium salt.

23. The process of Claim 3 wherein the compound of Formula I is:
methyl 2-[[[[(4-methoxy-6-methyl-1,3,5-triazin-2-yl)amino]carbonyl]amino]sulfonyl]benzoate, lithium salt.

24. The process of Claim 3 wherein the compound of Formula I is:
methyl 2-[[[[(4-methoxy-6-methyl-1,3,5-triazin-2-yl)amino]carbonyl]amino]sulfonyl]benzoate, sodium salt.

25. The process of Claim 3 wherein the compound of Formula I is:
methyl 2-[[[[(4-ethoxy-6-methylamino-1,3,5-triazin-2-yl)amino]carbonyl]amino]sulfonyl]benzoate, lithium salt.

26. The process of Claim 3 wherein the compound of Formula I is:
methyl 2-[[[[(4-ethoxy-6-methylamino-1,3,5-triazin-2-yl)amino]carbonyl]amino]sulfonyl]benzoate, sodium salt.

27. The process of Claim 3 wherein the compound of Formula I is:
ethyl-5-[[[[(4,6-dimethoxy-2-pyrimidinyl)amino]carbonyl]amino]sulfonyl]-1-methyl-1H-4-pyrazolecarboxylate, lithium salt.

28. The process of Claim 3 wherein the compound of Formula I is:
ethyl-5-[[[[(4,6-dimethoxy-2-pyrimidinyl)amino]carbonyl]amino]sulfonyl]-1-methyl-1H-4-pyrazolecarboxylate, sodium salt.

29. The process of Claim 3 wherein the compound of Formula I is:
2-[[[N-(4-methoxy-6-methyl-1,3,5-triazin-2-yl)N-amino]carbonyl]2-(2-methoxyethoxy)]benzenesulfonamide, lithium salt.

30. The process of Claim 3 wherein the compound of Formula I is:
2-[[[N-(4-methoxy-6-methyl-1,3,5-triazin-2-yl)N-amino]carbonyl]2-(2-methoxyethoxy)]benzenesulfonamide, sodium salt.

31. The process of Claim 9 wherein the solvent is triethylphosphate.

32. The process of Claim 10 wherein the solvent is 1-methyl-2-pyrrolidine.

33. The product of Claim 3.

34. The product of Claim 9.

35. The product of Claim 10.

European Patent Office

# EUROPEAN SEARCH REPORT

Application Number

EP 88 30 7616

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-4 599 412 (E.I. DU PONT DE NEMOURS & CO.)<br>* Example 8, lines 49-53; example 9 * | 3 | C 07 D 403/12<br>C 07 D 239/47<br>C 07 D 251/16<br>C 07 D 401/12<br>C 07 D 409/12<br>C 07 D 239/52<br>C 07 D 239/42<br>C 07 D 251/52<br>A 01 N 47/36 |
| A | US-A-3 033 902 (CHAS PFIZER & CO.)<br>* Column 3, lines 22-50; column 4, lines 29-43 * | 3 | |
| | | | **TECHNICAL FIELDS SEARCHED (Int. Cl.4)** |
| | | | C 07 D 403/00<br>C 07 D 239/00<br>C 07 D 251/00<br>C 07 D 401/00<br>C 07 D 409/00 |

The present search report has been drawn up for all claims

| Place of search | Date of completion of the search | Examiner |
|---|---|---|
| THE HAGUE | 22-11-1988 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document